# EUROPÄISCHE PATENTSCHRIFT

(11) **EP 1 627 874 B1**
(45) Veröffentlichungstag und Bekanntmachung des Hinweises auf die Patenterteilung: **02.04.2008**
(21) Anmeldenummer: 05016918.4
(22) Anmeldetag: 04.08.2005
(51) Int. Cl.: C07D 319/08

(54) **Herstellung von fluorierten 1,3-Benzodioxanen**
Preparation of fluorinated 1,3-benzodioxanes
Préparation de 1,3-benzodioxannes fluorés

(30) Priorität: 17.08.2004 DE 102004039876
(43) Veröffentlichungstag der Anmeldung: 22.02.2006
(73) Patentinhaber: LANXESS Deutschland GmbH, 51369 Leverkusen (DE)
(72) Erfinder: Joschek, Jens Peter, Dr., 51061 Köln (DE); Marhold, Albrecht, Dr., 51373 Leverkusen (DE); Pleschke, Axel, Dr., 51069 Köln (DE)

(56) Entgegenhaltungen:
- DE-A- 1 643 382

## Beschreibung

Die Erfindung betrifft neue fluorhaltige achirale 1,3-Benzodioxane sowie deren Herstellung.

Fluorhaltige 1,3-Benzodioxane, insbesondere die entsprechenden Aminobenzodioxane, sind wertvolle Zwischenverbindungen für die Synthese von life science Wirkstoffen, insbesondere von Pharmawirkstoffen in der Krebstherapie.

Bislang sind im Bereich der fluorierten 1,3-Benzodioxane lediglich Verbindungen bekannt, die entweder an einem der Methylenkohlenstoffe zwei verschiedene Substituenten tragen und/oder für die Verwendung als Bausteine für pharmazeutische Wirkstoffe keine ausreichende Lipophilie aufweisen (DE-A 16 43 382). Bei den Verbindungen, die ein Stereozentrum besitzen, ist eine aufwändige Enantiomerentrennung vor Weiterverwendung erforderlich.

Es bestand daher das Bedürfnis, das Problem der Enantiomerentrennung, das mit dem heutigen Stand der Technik nicht zufriedenstellend zu lösen ist, zu umgehen und für die Weiterverarbeitung zu pharmazeutischen Wirkstoffen geeignete fluorhaltige 1,3-Benzodioxane bereitzustellen.

Die Aufgabe, die der vorliegenden Erfindung zugrunde lag, bestand somit darin geeignete fluorhaltige 1,3-Benzodioxane bereitzustellen, bei denen keine aufwändige Enantiomerentrennung erforderlich ist und die beispielsweise zur Weiterverarbeitung zu pharmazeutischen Wirkstoffen für die Krebstherapie geeignet sind.

Überraschend konnte mit einer neuen Synthesestrategie, durch die Einführung eines zweiten identischen Substituenten an der Methylengruppe das Chiralitätsproblem umgangen und die Lipophilie in den Wirkstoffmolekülen merklich erhöht werden. Mit wenigen Syntheseschritten sind ein Vielzahl von Verbindungen der allgemeinen Formeln (I-a) oder (I-b) zu erhalten.

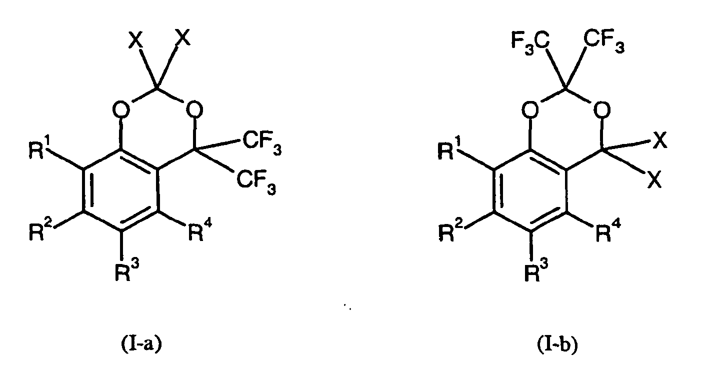

Gegenstand der vorliegenden Erfindung sind daher Verbindung der allgemeinen Formel (I-a) oder (I-b), 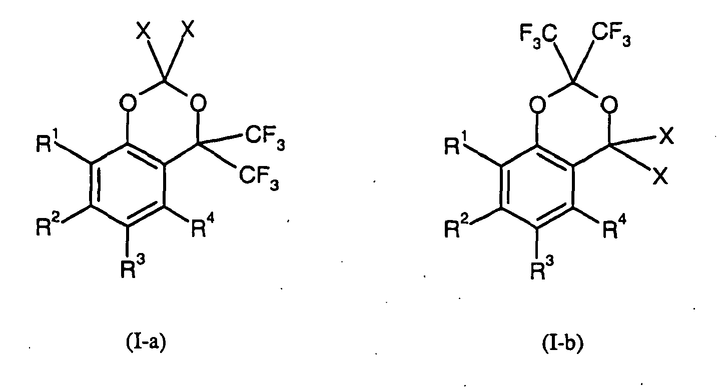 worin
- R¹, R², R³, R⁴: unabhängig voneinander für H, CN, NO₂, NH₂, OH, Halogen, lineare oder verzweigte gegebenenfalls teil- oder perfluorierte C₁-C₄-Alkylreste, lineare oder verzweigte gegebenenfalls teil- oder perfluorierte C₁-C₄-Alkoxyreste, CHO, COOH, COOR, SO₂CH₃, SO₂Hal, gegebenenfalls substituierte Phenyl- oder Pyridylreste, Fluorcarbonyl-, Benzoyl-, Trifluoracetyl-, Phenoxy-, Isocyanato-, SO₂F- und Difluorchlormethylreste stehen, wobei R für C₁-C₄-Alkyl und Hal für Halogen stehen,
- X: für H, Cl oder F, bevorzugt für F steht.

Bevorzugt stehen R¹, R², R³ und R⁴ unabhängig voneinander für H, CN, NO₂, NH₂, Br, CH₃, CF₃, OCH₃, OCF₃, CHO, COOH, COOR oder SO₂CH₃ stehen, wobei R für C₁-C₄-Alkylreste steht.

In bevorzugten Ausführungsformen der vorliegenden Erfindung ist wenigstens einer der Reste R¹, R², R³ und R⁴ von H verschieden.

In weiteren bevorzugten Ausführungsformen der vorliegenden Erfindung steht wenigstens einer der Reste R¹, R², R³ und R⁴ unabhängig voneinander für CN, NO₂, NH₂, Br, CHO, COOH, COOR oder SO₂CH₃, bevorzugt für NO₂, NH₂, wobei R für C₁-C₄-Alkylreste steht, und alle weiteren Reste R¹, R², R³ und R⁴ stehen unabhängig voneinander für H, CH₃, OCH₃, OCF₃ oder CF₃.

C₁-C₄-Alkyl bzw. C₁-C₄-Alkoxy steht im Rahmen der Erfindung - sofern nicht gesondert vermerkt -bevorzugt jeweils unabhängig für einen geradkettigen, verzweigten oder unverzweigten Alkyl- bzw. Alkoxy-Rest.

Beispielsweise steht C₁-C₄-Alkyl besonders bevorzugt für Methyl, Ethyl, n-Propyl, Isopropyl, n-Butyl oder tert.-Butyl.

Beispielsweise steht C₁-C₄-Alkoxy bevorzugt für Methoxy, Ethoxy, Isopropoxy, n-Propoxy, n-Butoxy oder tert.-Butoxy.

**Halogen** steht im Rahmen der Erfindung - sofern nicht gesondert vermerkt - für Fluor, Chlor, Brom oder Iod. Beispielsweise steht Halogen bevorzugt für Fluor, Chlor oder Brom, besonders bevorzugt für Fluor oder Chlor.

Als beispielhafte Verbindungen der erfindungsgemäßen 1,3-Benzodioxane seien die folgenden aufgeführt:

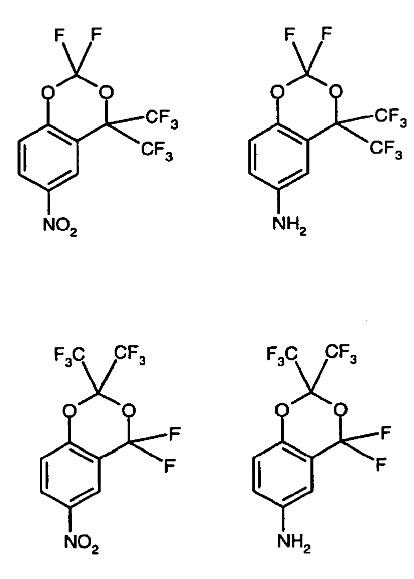

Die erfindungsgemäßen Verbindungen der allgemeinen Formel (I-a) oder (I-b) sind im Gegensatz zu den meisten bekannten Verbindungen achiral, so dass bei ihrer Herstellung das Problem der Enantiomerentrennung nicht auftritt. Daher können die erfindungsgemäßen Verbindungen auf einfachere Weise hergestellt werden als die bekannten chiralen Verbindungen.

Verbindungen der allgemeinen Formal (I-a) können zum Beispiel mit Umsetzung eines Phenols mit Hexafluoraceton zum Ketal beginnen, gefolgt von einer ringaufbauenden Reaktion mit Dihalogenmethanen, Formaldehyd oder anderen C₁-Bausteinen. Nachfolgende Chlorierung an der Methylengruppe und anschließende Fluorierung führen zur erfindungsgemäßen Verbindung, die dann gegebenenfalls am aromatischen Ring weiteren Substitutionen unterzogen werden kann. Auch weitere Reaktionen an den Substituenten am aromatischen Kern, wie beispielsweise Oxidationen, Reduktionen, Veresterungen oder Amidierungen etc. sind möglich.

Weiterhin Gegenstand der vorliegenden Erfindung ist daher ein Verfahren zur Herstellung einer erfindungsgemäßen Verbindung, wobei
a) eine Dihydroxyverbindung der allgemeinen Formel (II-a) 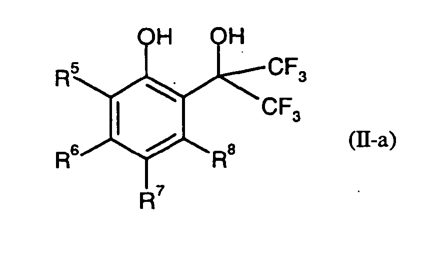 worin
   R⁵, R⁶, R⁷, R⁸ die oben für R¹, R², R³ und R⁴ genannte Bedeutung haben und
   mit einem gegebenenfalls substituierten Dihalogenmethan, Formaldehyd oder einem anderen C₁₋Baustein umgesetzt wird,
b) gegebenenfalls anschließend an der Methylengruppe chloriert wird, und
c) anschließend an der Methylengruppe fluoriert wird.

Die Ketale der allgemeinen Formel (I) können dabei - wie vorangehend bereits aufgeführt beispielsweise durch Umsetzung eines Phenols mit Hexafluoraceton hergestellt werden.

Verbindungen der allgemeinen Formal (I-b) können zum Beispiel ausgehend von Hydroxy- oder Halogenbenzylen, die in ortho-Position eine Hydroxygruppe oder ein Halogen tragen, durch die Umsetzung mit Hexafluoraceton und sich anschließender Ringschlussreaktion mit Säuren oder unter Buchwaldbedingungen, d.h. beispielsweise mit Palladiumdibenzylideneaceton und Tris(tert-butyl)phosphin als Katalysator in siedendem Toluol und Natrium-tert-butylat als Base, hergestellt werden. Die weitere Derivatisierung über Chlorierung und Chlor-Fluoraustausch erfolgt analog der Herstellung der Verbindungen der allgemeinen Formel (I-a).

Die Chlorierung kann beispielsweise mit einer Reihe von Chlorierungsmitteln, wie beispielsweise Chlor, PCl₃, PCl₅ und Kombination aus diesen, erfolgen. Bevorzugt erfolgt die Chlorierung jedoch mit Chlor, besonders bevorzugt unter Bestrahlung mit einer Hg-Dampflampe. Die Chlorierung erfolgt in Substanz oder in wenigstens einem Lösungsmittel, bei wenigstens einer Temperatur von ± 30°C oberhalb und unterhalb des Siedepunktes der Substanz oder der(s) gegebenenfalls verwendeten Lösungsmittel(s).

Als Lösungsmittel kommen bevorzugt solche in Frage, die unter den Bedingungen der Chlorierung gegenüber dem oder den Chlorierungsmitteln, insbesondere gegenüber Chlor inert sind. Dies sind beispielsweise halogenierte Lösungsmittel, wie z.B. Chlorbenzotrifluorid, Dichlorbenzotrifluorid.

Die Fluorierung kann mittels einer Reihe von Fluorierungsmitteln, wie beispielsweise HF, Alkali- und Übergangsmetallfluoriden sowie Hauptgruppenelementfluoriden, wie z.B. KF oder SbF₃, oder auch Ammoniumfluoriden des Typs R₄N⁺F, wobei R für C₁-C₄-Alkyl steht, erfolgen. Bevorzugt erfolgt die Fluorierung jedoch mit wasserfreier HF. Weiterhin bevorzugt wird die Fluorierung bei einer Temperatur von beispielsweise -10°C bis 20°C, besonders bevorzugt mit einem Überschuss wasserfreier HF durchgeführt.

Die aufgeführten Variationen von Substituenten und funktionellen Gruppen im aromatischen Ring des Benzodioxansystems können durch drei unterschiedliche Strategien erreicht werden. Zum einen kann das Phenoledukt mit unterschiedlichen Substituenten versehen sein, die die gesamte Synthese hindurch unverändert am Molekül bleiben und später gegebenenfalls in weitere Derivate überführt werden können. Um bei unsymmetrischen Startverbindungen eine Regioselektivität gewährleisten zu können, ist es gegebenenfalls sinnvoll, vor der Umsetzung mit Hexafluoraceton in ortho-Position reversible Schutzgruppen einzuführen. Als weitere Möglichkeit zur Erzielung eines bestimmten Substitutionsmusters am aromatischen Ring steht die elektrophile aromatische Substitution am fertigen fluorierten 1,3-Benzodioxansystem zur Verfügung, z.B. eine Nitrierung von der aus dann eine weitere Derivatisierung, beispielsweise über Reduktion zur Aminogruppe und nachfolgende Umsetzung zum Amid, möglich ist. Als dritte Möglichkeit besteht der nucleophile Angriff auf die fertigen fluorierten 1,3-Benzodioxansysteme z.B. mit Lithiumalkylen und der sich anschließenden klassischen Derivatisierung der metallorganischen Verbindungen.

Weiterhin Gegenstand der vorliegenden Erfindung ist daher ein Verfahren zur Herstellung der erfindungsgemäßen Verbindungen, wobei entweder nach vorangehend beschriebener Fluorierung oder in einem anderen Stadium des vorangehend beschriebenen Verfahrens zur Herstellung der erfindungsgemäßen Verbindungen eine Substitution am aromatischen Ring und/oder eine Reaktion an wenigstens einem der gegebenenfalls vorhandenen Substituenten am aromatischen Kern durchgeführt wird.

Weiterhin Gegenstand der vorliegenden Erfindung ist ein Verfahren zur Herstellung einer erfindungsgemäßen Verbindung, wobei eine Verbindung der allgemeinen Formel (I-a) oder (I-b) am aromatischen Ring substituiert und/oder eine Reaktion an wenigstens einem der gegebenenfalls vorhandenen Substituenten am aromatischen Kern durchgeführt wird.

Die erfindungsgemäßen Verbindungen eignen sich hervorragend zur Verwendung als Zwischenprodukte bzw. Bausteine in pharmazeutischen Wirkstoffen, insbesondere für die Krebstherapie.

### Beispiele:

### Beispiel 1:

a) Herstellung von 2-[2,2,2-trifluor-l-hydroxy-l-(trifluormethyl)ethyllphenol 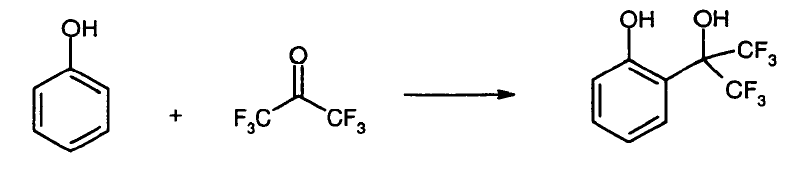 94,11 g (1000 mmol) Phenol und 166,02 g (100 mmol) AlCl₃ wurden in 1200 ml 1,2-Dichlorethan vorgelegt und auf - 35°C gekühlt. Bei dieser Temperatur wurde die vorausberechnete Menge von 166,02 g (1000 mmol) Hexafluoracteton aus einer Bombe zudosiert. Anschließend wurde der Reaktionsansatz auf Raumtemperatur (20°C, RT) kommen gelassen und für 36 h bei RT nachgerührt. Der Reaktionskolben wurde anschließend mit N₂ ausgeblasen und das Abgas in eine Waschflasche mit Wasser geleitet. Zur Aufarbeitung der verbleibenden Reaktionslösung wurden vorsichtig 500 ml Wasser zugegeben und gut nachgerührt. Anschließend wurde die organische Phase abgetrennt, die wässrige Phase mit CH₂Cl₂ extrahiert, mit Magnesiumsulfat getrocknet und das Lösungsmittel am Rotationsverdampfer entfernt. Dabei kristallisierte der Rückstand aus. Zur Reinigung wurde das erhaltene Kristallisat in n-Hexan aufgeschlämmt, abfiltriert und 230 g (842 mmol, Ausbeute: 82,4 % der Theorie) eines weißen, kristallinen Feststoffs erhalten.
b) Herstellung von 4,4-bis(trifluormethyl)-4H-1,3-benzodioxan 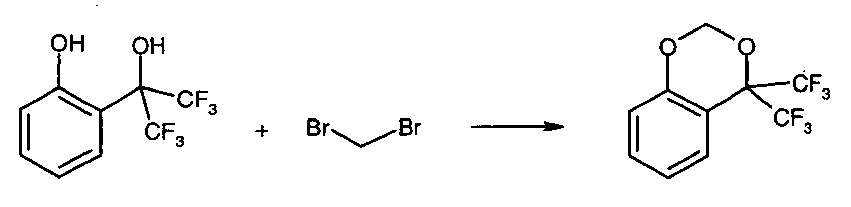 100,00 g (380 mmol) des Produktes aus Beispiel 1a) und 111,67 g (1920 mmol) Kaliumfluorid wurden in 1200 ml trockenem N,N-Dimethylformamid (DMF) vorgelegt und auf 130°C erhitzt. Während 75 min wurden 73,51 g (420 mmol) Dibrommethan zudosiert und 2 h nachgerührt. Per GC-Kontrolle wurde vollständiger Umsatz festgestellt. Die Reaktionsmischung wurde nach Abkühlen auf RT mit 3 l dest. H₂O versetzt. Anschließend wurde der Ansatz geteilt, jeweils mit 3 x 300 ml Methyl-*tert*-butyl-ether (MTBE) extrahiert, die organischen Phasen vereinigt, 1 x mit 100 ml 1 M wässriger NaOH gewaschen, 2 x mit 1 l dest. H₂O gewaschen, über Magnesiumsulfat getrocknet und das Lösungsmittel am Rotationsverdampfer entfernt. Es wurde eine rote Flüssigkeit erhalten. Nach Destillation über eine Brücke bei 6,7 mbar und einer Kopftemperatur von 68°C gingen 57 g (205 mmol, Ausbeute: 52,86 % der Theorie bei einer Reinheit von 98 %) des Produktes als klare Flüssigkeit über.
c) Herstellung von 2,2-Dichlor-4,4-bis(trifluormethyl)-4H-1,3-benzodioxan durch Chlorierung von 4,4-bis(trifluormethyl)-4H-1,3-benzodioxan 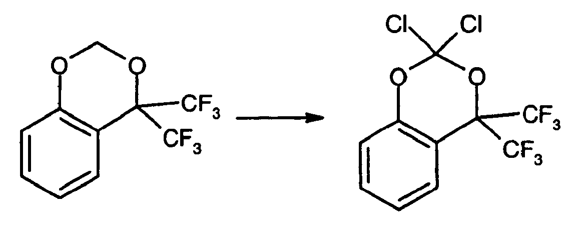 57 g (205 mmol) des Produktes aus Beispiel 1b) wurden vorgelegt, 5 g (37 mmol) PCl₃ zugegeben und bei 165°C für 6 Stunden unter Bestrahlung einer Hg-Dampflampe Chlor eingeleitet. Über eine kleine Brücke wurde bei 5,6-5 mbar, einer Badtemperatur von 130°C und einer Kopftemperatur von 80-85°C die Dichlorverbindung überdestilliert. Ausbeute: 34 g (99,7 mmol, 41,77 % der Theorie bei einer Reinheit von 86 %) farblose Flüssigkeit
d) Herstellung von 2,2-Difluor-4,4-bis(trifluormethyl)-4H-1,3-benzodioxan durch Fluorierung von 2,2-Dichlor-4,4-bis(trifluormethyl)-4H-1,3-benzodioxan 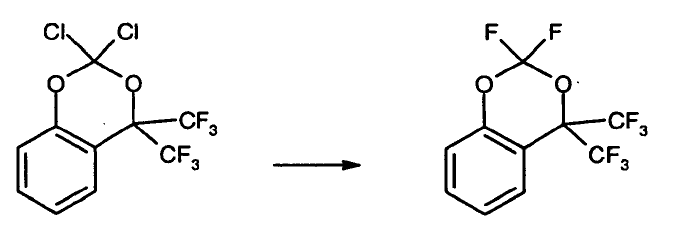 150 ml wasserfreie HF wurden bei - 4°C vorgelegt und bei - 5°C 34 g (99,70 mmol) Produkt aus Beispiel 1c) im Maße der HCl-Entwicklung zugetropft. Nach abklingender Gasentwicklung wurde die Temperatur auf RT ansteigen gelassen und 4 Stunden nachgerührt. Anschließend wurde die Reaktionsmischung auf ein Gemisch aus 350 g Eis und 650 g CH₂Cl₂ gegeben. Die wässrige und organische Phase wurden voneinander getrennt, die wässrige Phase 1 x mit 200 ml CH₂Cl₂ nachextrahiert, die organischen Phasen über Magnesiumsulfat getrocknet, und das Lösungsmittel am Rotationsverdampfer entfernt. 2,2-Difluor-4,4-bis(trifluormethyl)-4H-1,3-benzodioxan wurde in einer Ausbeute: 17 g (52 % der Theorie) in 95 %iger Reinheit erhalten.
e) Herstellung von 2,2-Difluor-4,4-bis(trifluormethyl)-4H-1,3-(6-nitrobenzo)-dioxan durch Nitrierung von 2,2-Difluor-4,4-bis(trifluormethyl)-4H-1,3-benzodioxan 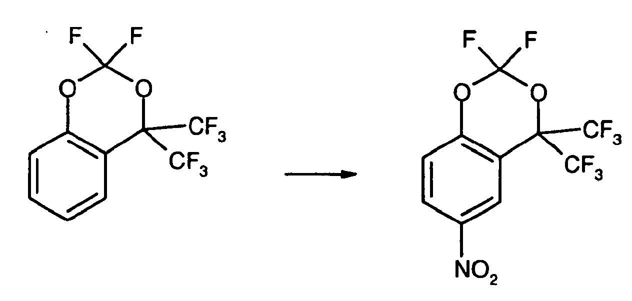 7,9 ml H₂SO₄ wurden vorgelegt und 6,5 ml HNO₃ unter Kühlung zugetropft. Bei 10°C wurden 17 g des Produkts aus Beispiel 1d) innerhalb von 5 Minuten zugetropft und 10 Minuten nachgerührt, wobei das nitrierte Zielprodukt ausfiel. Die Reaktionsmischung wurde auf 400 ml Eis gegeben, 3 x mit je 80 ml Essigsäureethylester extrahiert, 3 x mit 10 %iger NaHCO₃ gewaschen, über Magnesiumsulfat getrocknet und das Lösungsmittel entfernt. Ausbeute: 18 g gelber Feststoff (95 % der Theorie)
f) Herstellung von 2,2-Difluor-4,4-bis(trifluormethyl)-4H-1,3-(6-aminobenzo)-dioxan 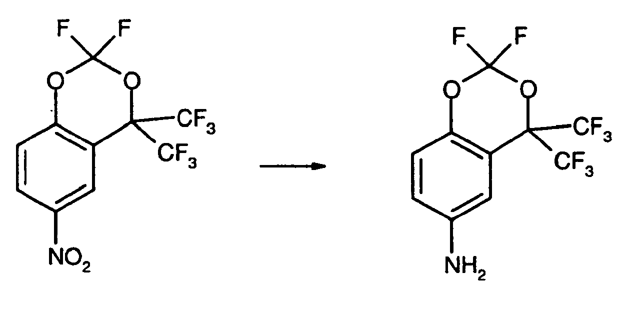 18 g des Produkts aus Beispiel 1e) wurden in 250 ml Essigsäure vorgelegt und bei Rückflusstemperatur (105°C Innentemperatur) innerhhalb von 2 Stunden 6,4 g Eisen zudosiert. Die Reaktionsmischung wurde anschließend auf 500 ml Eis gegeben, 3 x mit 300 ml Essigsäureethylester extrahiert, über Magnesiumsulfat getrocknet und vom Lösungsmittel befreit. Das Rohprodukt wurde über eine Minibrücke destilliert. Das Zielprodukt destilliert bei 0,1 mbar und einer Badtemperatur von 90- 110°C über. 2,2-Difluor-4,4-bis(trifluormethyl)-4H-1,3-benzodioxan-6-amino wurde mit einer Ausbeute von 60 % der Theorie erhalten.

## Patentansprüche

1. Verbindung der allgemeinen Formel (I-a) oder (I-b), 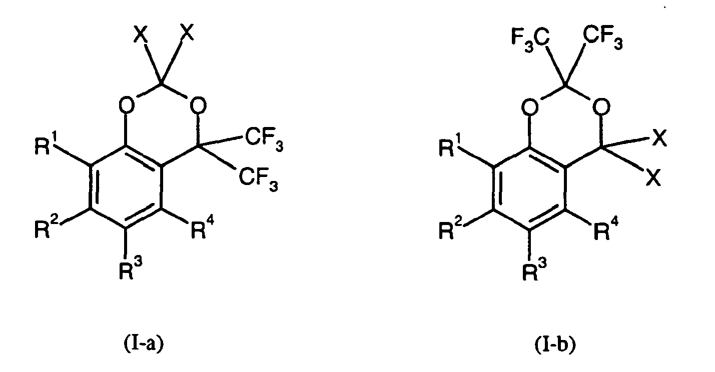 worin
R¹, R², R³, R⁴ unabhängig voneinander für H, CN, NO₂, NH₂, OH, Halogen, lineare oder verzweigte gegebenenfalls teil- oder perfluorierte C₁-C₄-Alkylreste, lineare oder verzweigte gegebenenfalls teil- oder perfluorierte C₁-C₄-Alkoxyreste, CHO, COOH, COOR, SO₂CH₃, SO₂Hal, gegebenenfalls substituierte Phenyl- oder Pyridylreste, Fluorcarbonyl-, Benzoyl-, Trifluoracetyl-, Phenoxy-, Isocyanato-, SO₂F- und Difluorchlormethylreste stehen, wobei R für C₁-C₄-Alkylreste und Hal für Halogenreste stehen,
X für H, Cl oder F steht.

2. Verbindung gemäß Anspruch 1, **dadurch gekennzeichnet, dass** X für F steht.

3. Verbindung gemäß Anspruch 1 oder 2, **dadurch gekennzeichnet, dass** R¹, R², R³ und R⁴ unabhängig voneinander für H, CN, NO₂, NH₂, Br, CH₃, CF₃, OCH₃, OCF₃, CHO, COOH, COOR oder SO₂CH₃ stehen, wobei R für C₁-C₄-Alkylreste steht.

4. Verbindung gemäß wenigstens einem der Ansprüche 1 bis 3, **dadurch gekennzeichnet, dass** wenigstens einer der Reste R¹, R², R³ und R⁴ von H verschieden ist.

5. Verbindung gemäß wenigstens einem der Ansprüche 1 bis 4, **dadurch gekennzeichnet, dass** wenigstens einer der Reste R¹, R², R³ und R⁴ unabhängig voneinander für CN, NO₂, NH₂, Br, CHO, COOH, COOR oder SO₂CH₃ stehen, wobei R für C₁-C₄-Alkylreste steht, und alle weiteren Reste R¹, R², R³ und R⁴ unabhängig voneinander für H, CH₃, OCH₃, OCF₃ oder CF₃ stehen.

6. Verfahren zur Herstellung einer Verbindung gemäß wenigstens einem der Ansprüche 1 bis 5, **dadurch gekennzeichnet, dass**
a) Eine Dihydroxyverbindung der allgemeinen Formel (II-a) 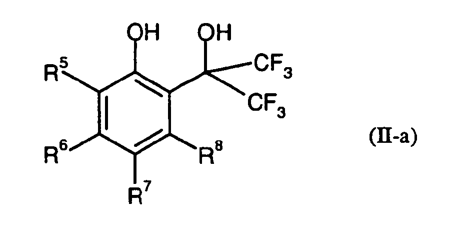 worin
R⁵, R⁶, R⁷, R⁸ die in Anspruch 1 für R¹, R², R³ und R⁴ genannte Bedeutung haben
mit einem gegebenenfalls substituierten Dihalogenmethan, Formaldehyd oder einem anderen C₁-Baustein umgesetzt wird,
b) gegebenenfalls anschließend an der Methylengruppe chloriert wird, und
c) anschließend an der Methylengruppe fluoriert wird.

7. Verfahren gemäß Anspruch 6, **dadurch gekennzeichnet, dass** die Chlorierung in wenigstens einem Lösungsmittel, bei wenigstens einer Temperatur von ± 30°C oberhalb und unterhalb des Siedepunktes dieser(s) Lösungsmittel(s) mit Chlor unter Bestrahlung erfolgt.

8. Verfahren gemäß Anspruch 6 oder 7, **dadurch gekennzeichnet, dass** die Fluorierung unter Verwendung von wasserfreier HF erfolgt.

9. Verfahren gemäß wenigstens einem der Ansprüche 6 bis 8, **dadurch gekennzeichnet, dass** die Fluorierung bei wenigstens einer Temperatur von -10°C bis 20°C mit einem Überschuss wasserfreier HF erfolgt.

10. Verfahren gemäß wenigstens einem der Ansprüche 6 bis 9, **dadurch gekennzeichnet, dass** R⁵, R⁶, R⁷ und R⁸ für H stehen.

11. Verfahren gemäß wenigstens einem der Ansprüche 6 bis 10, **dadurch gekennzeichnet, dass** anschließend eine Substitution am aromatischen Ring und/oder eine Reaktion an wenigstens einem der gegebenenfalls vorhandenen Substituenten am aromatischen Kern durchgeführt wird.

12. Verfahren zur Herstellung einer Verbindung gemäß wenigstens einem der Ansprüche 1 bis 5, **dadurch gekennzeichnet, dass** eine Verbindung gemäß wenigstens einem der Ansprüche 1 bis 5 am aromatischen Ring substituiert und/oder eine Reaktion an wenigstens einem der gegebenenfalls vorhandenen Substituenten am aromatischen Kern durchgeführt wird.

13. Verwendung einer Verbindung gemäß wenigstens einem der Ansprüche 1 bis 5 zur Herstellung von pharmazeutischen Wirkstoffen oder Arzneimitteln.

## Claims

1. Compound of the general formula (I-a) or (I-b), 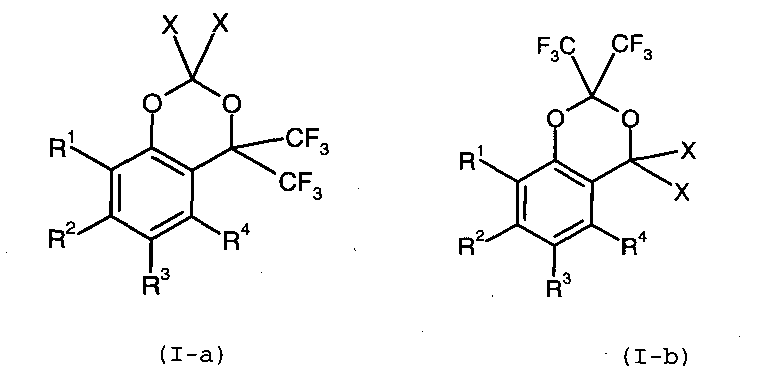 where
R¹, R², R³ , R⁴ are each independently H, CN, NO₂, NH₂, OH, halogen, linear or branched, optionally partly fluorinated or perfluorinated C₁-C₄-alkyl radicals, linear or branched, optionally partly fluorinated or perfluorinated C₁-C₄-alkoxy radicals, CHO, COOH, COOR, SO₂CH₃, SO₂Hal, optionally substituted phenyl or pyridyl radicals, fluorocarbonyl, benzoyl, trifluoroacetyl, phenoxy, isocyanato, SO₂F and difluorochloromethyl radicals, where R is a C₁-C₄-alkyl radical and Hal is a halogen radical,
X is H, Cl or F.

2. Compound according to Claim 1, **characterized in that** X is F.

3. Compound according to Claim 1 or 2, **characterized in that** R¹, R², R³ and R⁴ are each independently H, CN, NO₂, NH₂, Br, CH₃, CF₃, OCH₃, OCF₃, CHO, COOH, COOR or SO₂CH₃, where R is a C₁-C₄-alkyl radical.

4. Compound according to at least one of Claims 1 to 3, **characterized in that** at least one of the R¹, R², R³ and R⁴ radicals is different to H.

5. Compound according to at least one of Claims 1 to 4, **characterized in that** at least one of the R¹, R², R³ and R⁴ radicals is independently CN, NO₂, NH₂, Br, CHO, COOH, COOR or SO₂CH₃, where R is a C₁-C₄-alkyl radical, and all further R¹, R², R³ and R⁴ radicals are each independently H, CH₃, OCH₃, OCF₃ or CF₃.

6. Process for preparing a compound according to at least one of Claims 1 to 5, **characterized in that**
a) a dihydroxy compound of the general formula (II-a) 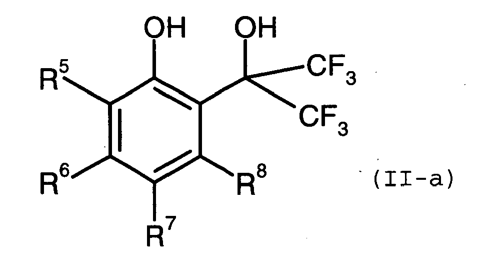 where
R⁵, R⁶, R⁷, R⁸ are each as defined for R¹, R², R³ and R⁴ in Claim 1
is reacted with an optionally substituted dihalomethane, formaldehyde or another C₁ unit,
b) is optionally chlorinated subsequently on the methylene group, and
c) is subsequently fluorinated on the methylene group.

7. Process according to Claim 6, **characterized in that** the chlorination is effected under irradiation with chlorine in at least one solvent at at least one temperature of ± 30°C above and below the boiling point of this/these solvent(s).

8. Process according to Claims 6 or 7, **characterized in that** the fluorination is effected using anhydrous HF.

9. Process according to at least one of Claims 6 to 8, **characterized in that** the fluorination is effected with an excess of anhydrous HF at at least one temperature of -10°C to 20°C.

10. Process according to at least one of Claims 6 to 9, **characterized in that** R⁵, R⁶, R⁷ and R⁸ are each H.

11. Process according to at least one of Claims 6 to 10, **characterized in that** a substitution is subsequently carried out on the aromatic ring and/or a reaction on at least one of any substituents present on the aromatic ring.

12. Process for preparing a compound according to at least one of Claims 1 to 5, **characterized in that** a compound according to at least one of Claims 1 to 5 is substituted on the aromatic ring and/or a reaction is carried out on at least one of any substituents present on the aromatic ring.

13. Use of a compound according to at least one of Claims 1 to 5 for producing active pharmaceutical ingredients or medicaments.

## Revendications

1. Composé de formule générale (I-a) ou (I-b), 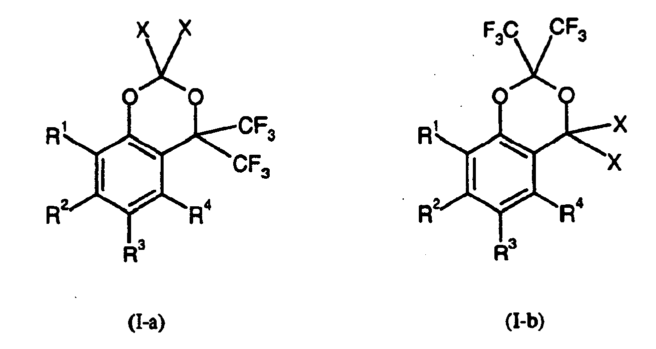 dans laquelle :
R¹, R², R³, R⁴ représentent indépendamment les uns des autres H, CN, NO₂, NH₂, OH, un halogène, des radicaux alkyle en C₁-C₄ linéaires ou ramifiés, éventuellement perfluorés ou partiellement fluorés, des radicaux alcoxy en C₁-C₄ linéaires ou ramifiés, éventuellement perfluorés ou partiellement fluorés, CHO, COOH, COOR, SO₂CH₃, SO₂Hal, des radicaux phényle ou pyridyle éventuellement substitués, des radicaux fluorocarbonyle, benzoyle, trifluoroacétyle, phénoxy, isocyanato, SO₂F et difluorochlorométhyle, R représentant des radicaux alkyle en C₁-C₄ et Hal représentant des radicaux halogène,
X représente H, Cl ou F.

2. Composé selon la revendication 1, **caractérisé en ce que** X représente du fluor.

3. Composé selon la revendication 1 ou 2, **caractérisé en ce que** R¹, R² R³ et R⁴ représentent indépendamment les uns des autres H, CN, NO₂, NH₂, Br, CH₃, CF₃, OCH₃, OCF₃ , CHO, COOH , COOR ou SO₂CH₃, R représentant des radicaux alkyle en C₁-C₄.

4. Composé selon au moins l'une quelconque des revendications 1 à 3, **caractérisé en ce qu'**au moins un des radicaux R¹, R², R³ et R⁴ est différent de H.

5. Composé selon au moins l'une quelconque des revendications 1 à 4, **caractérisé en ce qu'**au moins un des radicaux R¹, R², R³ et R⁴ représente indépendamment des autres CN, NO₂, NH₂, Br, CHO, COOH, COOR ou SO₂CH₃, R représentant des radicaux alkyle en C₁-C₄ et tous les autres radicaux R¹, R², R³ et R⁴ représentant indépendamment les uns des autres H, CH₃, OCH₃, OCF₃ ou CF₃.

6. Procédé de préparation d'un composé selon l'une quelconque des revendications 1 à 5, **caractérisé en ce que** :
a) un composé dihydroxy de formule générale (II-a) 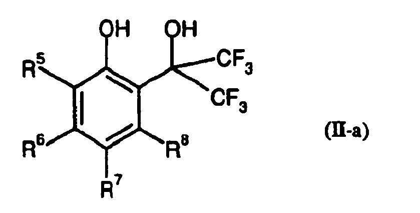 dans laquelle :
R⁵, R⁶, R⁷, R⁸ ont la signification donnée dans la revendication 1 pour R¹, R², R³ et R⁴ est amené à réagir avec un formaldéhyde ou un dihalogénométhane éventuellement substitué, ou un autre composant Ci,
b) est éventuellement chloré par la suite sur le groupe méthylène, et
c) est ensuite fluoré sur le groupe méthylène.

7. Procédé selon la revendication 6, **caractérisé en ce que** la chloration a lieu dans au moins un solvant, à au moins une température de ± 30°C supérieure ou inférieure au point d'ébullition de ce(s) solvant(s), avec du chlore et sous irradiation.

8. Procédé selon la revendication 6 ou 7, **caractérisé en ce que** la fluoration a lieu en utilisant du HF anhydre.

9. Procédé selon au moins l'une quelconque des revendications 6 à 8, **caractérisé en ce que** la fluoration a lieu à au moins une température de -10°C à 20°C avec un excédent de HF anhydre.

10. Procédé selon au moins l'une quelconque des revendications 6 à 9, **caractérisé en ce que** R⁵, R⁶, R⁷ et R⁸ représentent de l'hydrogène.

11. Procédé selon au moins l'une quelconque des revendications 6 à 10, **caractérisé en ce qu'**une substitution est ensuite mise en oeuvre sur le cycle aromatique et/ou une réaction est mise en oeuvre sur au moins un des substituants éventuellement présents sur le noyau aromatique.

12. Procédé de préparation d'un composé selon au moins l'une quelconque des revendications 1 à 5, **caractérisé en ce qu'**un composé selon au moins l'une quelconque des revendications 1 à 5 est substitué sur le cycle aromatique et/ou une réaction est mise en oeuvre sur au moins un des substituants éventuellement présents sur le noyau aromatique.

13. Utilisation d'un composé selon au moins l'une quelconque des revendications 1 à 5 pour la préparation de substances actives pharmaceutiques ou de médicaments.
